# EUROPÄISCHE PATENTSCHRIFT

(11) **EP 1 357 372 B1**
(45) Veröffentlichungstag und Bekanntmachung des Hinweises auf die Patenterteilung: **08.01.2014**
(21) Anmeldenummer: 03009109.4
(22) Anmeldetag: 19.04.2003
(51) Int. Cl.: A61M 1/36, A61M 5/168, G01L 9/00

(54) **Drucksensor**
Pressure sensor
Capteur de pression

(30) Priorität: 24.04.2002 DE 20206474 U
(43) Veröffentlichungstag der Anmeldung: 29.10.2003
(73) Patentinhaber: B. Braun Melsungen AG, 34212 Melsungen (DE)
(72) Erfinder: Steger, Jürgen, 34327 Körle (DE)
(74) Vertreter: von Kreisler Selting Werner

(56) Entgegenhaltungen:
- WO-A-98/36254
- DE-A- 2 420 610
- DE-A- 4 013 403
- DE-C1- 3 838 689
- DE-U1- 29 602 065
- US-A- 4 369 780
- US-A- 4 702 675
- US-A- 5 031 460

## Beschreibung

Die Erfindung betrifft einen Drucksensor mit einem Schlauch zum Hindurchleiten eines Fluides, zwei den Querschnitt des Schlauches deformierenden Stützkörpern und einem auf den Innendruck des Schlauches reagierenden Kraftsensor.

In der Medizintechnik werden Schläuche verwendet, um durch Infusion einem Patienten Flüssigkeit zuzuführen oder um einem Patienten Flüssigkeit abzunehmen oder um Flüssigkeit zwischen Geräten bzw. Maschinen zu transportieren. Dabei besteht die Notwendigkeit, einen Schlauchverschluss, wie er beispielsweise durch Abknicken des Schlauchs auftritt, zu erkennen. Außerdem ist es häufig notwendig, den Innendruck eines Schlauchs zu begrenzen.

Aus DE 40 13 403 C2 ist ein Drucksensor bekannt, bei dem der Schlauch zwischen zwei Stützkörpern zusammengedrückt wird. Der eine Stützkörper bildet ein ortsfestes Widerlager und der andere Stützkörper ist beweglich und er wird von einem Kraftsensor abgestützt. Der Kraftsensor ermittelt die auf den Schlauch einwirkende Kraft. Dieser Kraft wirken die Rückstellkraft des Schlauchs und der Innendruck des Schlauchs entgegen. Um den Innendruck mit hinreichender Genauigkeit zu messen, muss der verwendete Schlauch und seine Rückstellfähigkeit bekannt sein. Zur Ermittlung der Materialeigenschaften des Schlauchs erfolgt eine zeitaufwendige Vergleichsmessung. Allerdings sind die Materialeigenschaften des Schlauchs in der Regel von der Temperatur abhängig, so dass bei unterschiedlichen Betriebstemperaturen unterschiedliche Vergleichsmessungen durchgeführt werden müssten. Die zur Deformierung des Schlauchs benötigte Verformungskraft, die insbesondere in den Randzonen des Schlauchs hoch ist, überlagert das Drucksignal bis zum Faktor 10. Daher sind Kombinationsmessungen beider Kräfte stark fehlerbehaftet. Zudem ist die Verformungskraft nicht zeitkonstant über die Benutzungsdauer des Schlauchs.

Ein ähnlicher Drucksensor, bei dem zunächst eine Messung der Schlaucheigenschaften erfolgt, bevor die Signale des Kraftsensors ausgewertet werden, ist beschrieben in DE 38 38 689 C1. Auch hier sind die Rückstellkräfte des Schlauchs mit der durch den Schlauchinnendruck erzeugten Kraft überlagert.

In DE 296 02 065 U1 ist ein Drucksensor beschrieben, bei dem der Schlauch in einen Stützkörper eingelegt ist, der die Form eines runden Messrohres hat. Daher wird der Schlauch in seiner ursprünglichen Form abgestützt, wobei er durch den Stützkörper nicht flachgedrückt wird. Der Stützkörper bzw. das Messrohr hat einen langgestreckten Schlitz, in den ein Steg eines Stößels hineinragt, welcher von einem Kraftsensor abgestützt ist. Der Steg drückt gegen den Schlauch und bewirkt eine örtliche Deformierung des Querschnitts des im übrigen rund bleibenden Schlauchs.

Ein Drucksensor, von dem der Oberbegriff des Patentanspruchs 1 ausgeht, ist beschrieben in WO 98/36254. Bei diesem Drucksensor wird der Schlauch zwischen zwei Stützkörpern so deformiert, dass er eine ebene Seitenwand erhält. In dieser befindet sich eine Drucktastöffnung, durch die ein Stift hindurchragt. Der runde Stift überträgt Bewegungen der Schlauchwand, die durch den Druck hervorgerufen werden auf einen Kraftsensor.

Der Erfindung liegt die Aufgabe zugrunde, Drucksensoren zu schaffen, dessen Messsignale von dem verwendeten Schlauch und dessen Einlegedauer weitgehend unabhängig sind.

Die erfindungsgemäßen Drucksensoren sind durch die Patentansprüche 1 und 4 definiert. Bei diesen Drucksensoren sind die Stützkörper plattenförmig und sie haben jeweils eine plane Auflagefläche. Das Kraftübertragungsmittel ist ein Steg oder ein inkompressibles Gel, der oder das sich in Längsrichtung des Schlauchs erstreckt und in einem in Längsrichtung des Schlauchs verlaufenden Spalt angeordnet ist, der durch den Stützkörper hindurchgeht.

Das Kraftübertragungsmittel sollte einen möglichst großen Abstand von den beiden Biegebereichen des Schlauchs haben. Insbesondere hat es eine Breite, die maximal 25 % der lichten Weite (Breite) des durch die Stützkörper deformierten Schlauchs beträgt, und insbesondere maximal 15 %.

Dadurch wird erreicht, dass die Deformationskräfte des Schlauchs von den Stützkörpern aufgenommen und von dem Kraftsensor im Wesentlichen ferngehalten werden. Der Kraftsensor ist von den Stützkörpern entkoppelt, d.h. er reagiert nicht auf Kräfte, die durch eine Belastung der Stützkörper erzeugt werden. Die Stützkörper bewirken insbesondere die Abstützung in den beiden Rundungsbereichen des flachgedrückten Schlauchs. Das Kraftübertragungsmittel überträgt die Kraft von dem Mittelbereich des flachgedrückten Schlauchs auf den Kraftsensor. Hierbei wirken sich nur Verformungen des Mittelbereichs aus, die durch den Innendruck des Schlauchs hervorgerufen werden.

Vorzugsweise ist der Kraftsensor ein Sensor mit besonders geringer Auslenkung, wobei die maximale Auslenkung weniger als 1 mm beträgt. Dadurch wird die durch den Innendruck des Schlauchs hervorgerufene Zusatzdeformierung kleingehalten und der Einfluss des Schlauchmaterials auf das Messergebnis wird begrenzt.

Zur Abstützung des Kraftübertragungsmittels können mehrere Kraftsensoren vorgesehen sein, die in Längsrichtung des Schlauchs verteilt angeordnet sind.

Das Kraftübertragungsmittel besteht aus einem in Längsrichtung des Schlauchs verlaufenden Festkörpersteg oder aus einem inkompressiblen Gel. Wichtig ist, dass der Steg relativ zu dem Stützkörper, durch den er sich erstreckt, bewegbar ist und im Mittelbereich des flachgedrückten Schlauches angreift, so dass er von den Kräften, die durch das Zusammendrücken des Schlauches entstehen, weitgehend befreit ist.

Die Stützkörper haben plane Auflageflächen.

Da die Verformungskräfte von den Stützkörpern aufgenommen werden, ruht der mittlere Bereich des Schlauches wie eine Membran auf dem Kraftübertragungsmittel. Wird in dem Schlauch Druck erzeugt, so überträgt sich die der Querschnittsfläche des Spaltes proportionale Druckkraft über den Steg auf den Kraftsensor. Aus der Querschnittsfläche A des Steges und der Kraft F lässt sich der Druck P im Schlauch berechnen zu P = F x A. Die Auswertung des Kraftsignals erfolgt in einem Mikroprozessor.

Im Folgenden werden unter Bezugnahme auf die Zeichnungen Ausführungsbeispiele der Erfindung näher erläutert.

Es zeigen:
- Fig. 1: eine schematische perspektivische Schnittdarstellung einer ersten Ausführungsform des Drucksensors mit einem Festkörpersteg, und
- Fig. 2: gleiche Darstellung wie Figur 1 einen Drucksensor mit einem inkompressiblen Gel.

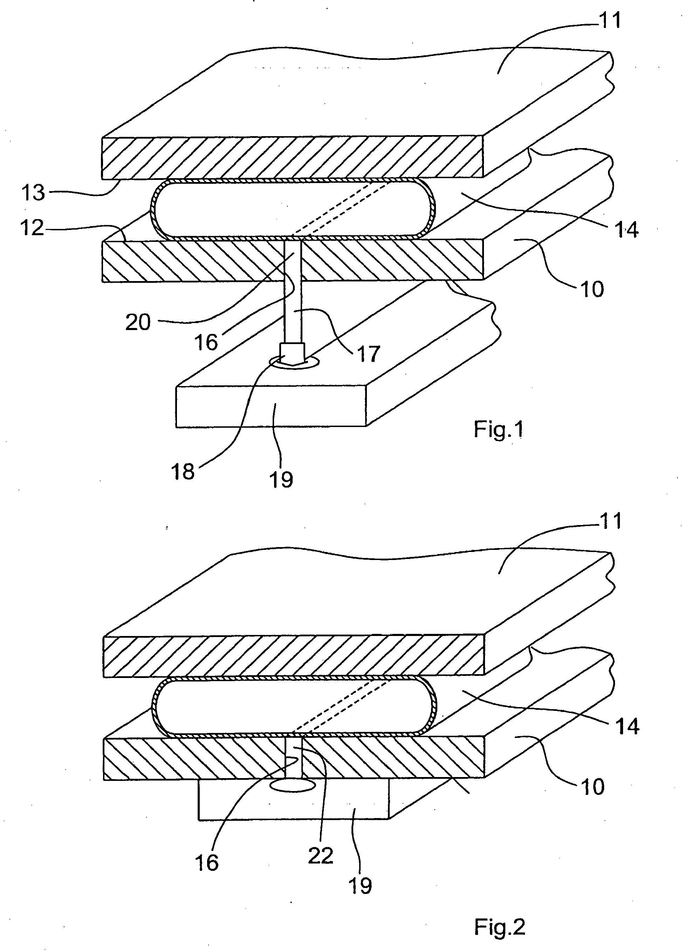

Der Drucksensor nach Figur 1 weist zwei plattenförmige Stützkörper 10,11 auf, die jeweils eine plane Auflagefläche 12,13 haben. Zwischen den parallelen Auflagefläche 12,13 wird der Schlauch 14 flachgedrückt. Hierzu ist einer der Stützkörper 10,11 in Richtung auf den anderen Stützkörper bewegbar.

Der Schlauch 14 besteht aus einem flexiblen Material, das elastisch ist und somit eine Rückstellfähigkeit hat. Der Querschnitt des Schlauchs 14 ist im unkomprimierten Zustand rund. Im flachgedrückten Zustand des Schlauchs hat der Schlauchquerschnitt eine längliche Form, so wie dies in Figur 1 dargestellt ist. In dem Schlauchlumen 15 befindet sich eine Flüssigkeit, beispielsweise eine Infusionslösung, die einem Patienten zugeführt wird.

Durch den Stützkörper 10 erstreckt sich ein längslaufender Spalt 16, der im Mittelbereich des flachgedrückten Schlauchs 14 angeordnet ist. Der Schlauch 14 wird durch (nicht dargestellte) Seitenwände in einer definierten Position zwischen den Stützkörpern 10,11 gehalten. Der Spalt 16 erstreckt sich durch die Dicke des Stützkörpers 10 hindurch. In dem Spalt 16 ist ein Kraftübertragungsmittel in Form eines Steges 17 angeordnet. Der Steg 17 ist quer zu dem Stützkörper 10 verschiebbar. Er erstreckt sich in Längsrichtung des Schlauchs 14. An dem einen Ende des Steges 17 ist ein Fuß 18 vorgesehen, der gegen einen Kraftsensor 19 drückt. Der Kraftsensor 19 ist an dem Stützkörper 10 durch (nicht dargestellte) Zuhaltemittel starr befestigt. Der Kraftsensor 19 weist beispielsweise Dehnmessstreifen auf, die zu einer Brückenschaltung geschaltet sind und ein elektrisches Signal erzeugen, das der auf den Kraftsensor 19 einwirkenden Kraft F proportional ist.

Ein entsprechender Kraftsensor 19 kann auch an dem gegenüberliegenden Ende des Steges 17 angeordnet sein, wo ein dem Fuß 18 entsprechender weiterer Fuß vorgesehen ist.

Der Steg 17 ist ein Festkörper, der beispielsweise aus einer starren Platte besteht. Das obere Ende 20 des Steges 17 drückt unmittelbar gegen den Umfang des Schlauchs 14, und zwar im Mittelbereich des zwischen den Stützkörpern 10,11 flachgedrückten Schlauchs, wo die Schlauchwandung geradlinig verläuft.

Bei dem Ausführungsbeispiel von Figur 2 ist der eine Stützkörper 10 ebenfalls mit einem längslaufenden Spalt 16 versehen und an der dem Schlauch 14 abgewandten Seite des Stützkörpers 10 ist ein den Spalt 16 bedeckender Kraftsensor 19 befestigt. Der Spalt 16 ist mit einem Kraftübertragungsmittel gefüllt, das aus einem inkompressiblen Gel 22 besteht. Dieses Gel 22 drückt an einem Ende gegen den geraden Teil der Wandung des flachgedrückten Schlauchs 14 und am anderen Ende gegen die druckempfindliche Fläche des Kraftsensors 19. Das Gel 22 kann durch verformbare Membranen eingeschlossen sein, die den Spalt 16 an dessen Oberseite und an dessen Unterseite bedecken. Das Gel 22 bildet ein Kraftübertragungsmittel, das die innendruckabhängige Verformung der Schlauchwandung auf den Kraftsensor 19 überträgt.

In beiden Fällen wird bei einer Druckänderung im Schlauch 14 nur die Schlauchverformung am Spalt 16 zur Erzeugung des Kraftsignals ausgewertet. Der Kraftsensor 19 erzeugt ein elektrisches Signal, welches dem Innendruck des Schlauchs mit hoher Genauigkeit proportional ist.

Bei einem Ausführungsbeispiel der Erfindung entspricht ein Druck von 1 bar einer Kraft von 100.000 N/m². Bei einer Fläche des Spalts von 10 mm x 2 mm ergeben sich 2 N/bar. Der Proportionalitätsfaktor beträgt hierbei 0,5.

## Patentansprüche

1. Drucksensor mit einem Schlauch (14) zum Hindurchleiten eines Fluides, zwei den Querschnitt des Schlauchs (14) deformierenden Stützkörpern (10,11) und einem auf den Innendruck des Schlauchs reagierenden Kraftsensor (19),
wobei mindestens einer der Stützkörper (10,11) ein Kraftübertragungsmittel enthält, das sich über weniger als 25 % der lichten Weite des von den Stützkörpern deformierten Schlauchs erstreckt und relativ zu diesem Stützkörper bewegbar ist und mit einem Ende gegen den Schlauch (14) und mit dem anderen Ende gegen den Kraftsensor (19) drückt,
wobei die Stützkörper (10,11) plattenförmig sind und jeweils eine plane Auflagefläche (12,13) haben und
**dadurch gekennzeichnet,**
**dass** das Kraftübertragungsmittel ein Steg (17) ist, der sich in Längsrichtung des Schlauchs (14) erstreckt und in einem in Längsrichtung des Schlauchs (14) verlaufenden Spalt (16), der durch den Stützkörper (10) hindurchgeht, angeordnet ist.

2. Drucksensor nach Anspruch 1, **dadurch gekennzeichnet, dass** der Kraftsensor (19) eine maximale Auslenkung von weniger als 1 mm hat.

3. Drucksensor nach Anspruch 1 oder 2, **dadurch gekennzeichnet, dass** der Steg (17) aus einer starren Platte besteht.

4. Drucksensor mit einem Schlauch (14) zum Hindurchleiten eines Fluides, zwei den Querschnitt des Schlauchs (14) deformierenden Stützkörpern (10,11) und einem auf den Innendruck des Schlauchs reagierenden Kraftsensor (19),
wobei mindestens einer der Stützkörper (10,11) ein Kraftübertragungsmittel enthält, das sich über weniger als 25 % der lichten Weite des von den Stützkörpern deformierten Schlauchs erstreckt und relativ zu diesem Stützkörper bewegbar ist und mit einem Ende gegen den Schlauch (14) und mit dem anderen Ende gegen den Kraftsensor (19) drückt,
wobei die Stützkörper (10,11) plattenförmig ohne seitliche Schlauchdeformationswände sind und jeweils eine plane Auflagefläche (12,13) haben und
**dadurch gekennzeichnet,**
**dass** das Kraftübertragungsmittel aus einem inkompressiblen Gel (22) besteht, das sich in Längsrichtung des Schlauchs (14) erstreckt und in einem in Längsrichtung des Schlauchs (14) verlaufenden Spalt (16), der durch den Stützkörper (10) hindurchgeht, angeordnet ist.

## Claims

1. A pressure sensor comprising a hose (14) for passing a fluid, two supporting bodies (10,11) deforming the cross-section of the hose (14), and a force sensor (19) reacting to the internal pressure of the hose,
wherein at least one of the supporting bodies (10,11) includes a force transmission means which extends over less than 25% of the lumen of the hose deformed by the supporting bodies, and which is movable relative to this supporting body and presses against the hose (14) with one end and against the force sensor (19) with the other end,
wherein the supporting bodies (10, 11) are plate-shaped and each have a planar contact surface (12, 13),
**characterized in that**
the force transmission means is a web (17) extending in the longitudinal direction of the hose (14) and arranged in a gap (16) that extends in the longitudinal direction of the hose (14) and passes through the supporting body (10).

2. The pressure sensor according to claim 1, **characterized in that** the force sensor (19) has a maximum deflection of less than 1 mm.

3. The pressure sensor according to claim 1 or 2, **characterized in that** the web (17) is formed by a rigid plate.

4. A pressure sensor comprising a hose (14) for passing a fluid, two supporting bodies (10,11) deforming the cross-section of the hose (14), and a force sensor (19) reacting to the internal pressure of the hose,
wherein at least one of the supporting bodies (10,11) includes a force transmission means which extends over less than 25% of the lumen of the hose deformed by the supporting bodies, and which is movable relative to this supporting body and presses against the hose (14) with one end and against the force sensor (19) with the other end,
wherein the supporting bodies (10, 11) are plate-shaped without any lateral hose deforming walls and each have a planar contact surface (12, 13),
**characterized in that**
the force transmission means is an incompressible gel (22) extending in the longitudinal direction of the hose (14) and arranged in a gap (16) that extends in the longitudinal direction of the hose (14) and passes through the supporting body (10).

## Revendications

1. Capteur de pression comportant un tuyau flexible (14) pour la circulation d'un fluide, deux corps d'appui (10, 11) déformant la section transversale du tuyau flexible (14), et un capteur de force (19) réagissant à la pression intérieure dudit tuyau flexible,
au moins un des corps d'appui (10, 11) contenant un moyen de transmission de force, qui s'étend sur moins de 25 % de la largeur intérieure du tuyau flexible déformé par les corps d'appui et qui est mobile par rapport à ce corps d'appui et pousse avec une extrémité contre le tuyau flexible (14) et avec l'autre extrémité contre le capteur de force (19),
les corps d'appui (10, 11) étant réalisés en forme de plaques et étant munis chacun d'une surface d'appui (12, 13) plane, et
**caractérisé en ce que**
le moyen de transmission de force est une traverse (17) qui s'étend dans le sens longitudinal du tuyau flexible (14) et est disposée dans une fente (16), orientée dans le sens longitudinal du tuyau flexible (14) et traversant le corps d'appui (10).

2. Capteur de pression selon la revendication 1, **caractérisé en ce que** le capteur de force (19) a une flèche maximale de moins de 1 mm.

3. Capteur de pression selon la revendication 1 ou 2, **caractérisé en ce que** la traverse (17) est formée par une plaque rigide.

4. Capteur de pression comportant un tuyau flexible (14) pour la circulation d'un fluide, deux corps d'appui (10, 11) déformant la section transversale du tuyau flexible (14), et un capteur de force (19) réagissant à la pression intérieure dudit tuyau flexible,
au moins un des corps d'appui (10, 11) contenant un moyen de transmission de force, qui s'étend sur moins de 25 % de la largeur intérieure du tuyau flexible déformé par les corps d'appui et qui est mobile par rapport à ce corps d'appui et pousse avec une extrémité contre le tuyau flexible (14) et avec l'autre extrémité contre le capteur de force (19),
les corps d'appui (10, 11) étant réalisés en forme de plaques et étant munis chacun d'une surface d'appui (12, 13) plane, et
**caractérisé en ce que**
le moyen de transmission de force est formé par un gel (22) incompressible, qui s'étend dans le sens longitudinal du tuyau flexible (14) et est disposé dans une fente (16), orientée dans le sens longitudinal du tuyau flexible (14) et traversant le corps d'appui (10).
